(19) Europäisches Patentamt
European Patent Office
Office européen des brevets

(11) **EP 1 752 152 A1**

(12) **EUROPEAN PATENT APPLICATION**
published in accordance with Art. 158(3) EPC

(43) Date of publication:
**14.02.2007 Bulletin 2007/07**

(51) Int Cl.:
*A61K 31/718* (2006.01)   *A61K 31/722* (2006.01)
*A61K 31/765* (2006.01)   *A61K 45/00* (2006.01)
*A61P 27/02* (2006.01)   *A61P 27/12* (2006.01)

(21) Application number: **05743700.6**

(22) Date of filing: **30.05.2005**

(86) International application number:
**PCT/JP2005/009846**

(87) International publication number:
**WO 2005/115411 (08.12.2005 Gazette 2005/49)**

(84) Designated Contracting States:
**AT BE BG CH CY CZ DE DK EE ES FI FR GB GR HU IE IS IT LI LT LU MC NL PL PT RO SE SI SK TR**

(30) Priority: **31.05.2004   JP 2004161147**

(71) Applicant: **Senju Pharmaceutical Co., Ltd.
Osaka-shi, Osaka 541-0046 (JP)**

(72) Inventors:
• **MATSUHISA, Keiichi
  6710221 (JP)**
• **INOUE, Yutaka
  6650861 (JP)**

(74) Representative: **Behnisch, Werner et al
Reinhard-Skuhra-Weise & Partner GbR,
Friedrichstrasse 31
80801 München (DE)**

(54) **AGENT FOR VISUALIZING TRANSPARENT TISSUE**

(57)   Described is a highly safe means to improve visibility of transparent tissues of the eye, i.e., the vitreous body, the lens or the cornea, in a surgical operation on them. The means comprises fine particles of a macromolecular compound, in particular, those one gram of which cannot be dissolved in less than 30 mL of water within 30 minutes at 20 °C, and whose average particle size is 0.01-500 μm, and which, among others, is a biodegradable macromolecular compound and/or a water soluble macromolecular compound.

Fig. 1

EP 1 752 152 A1

**Description**

TECHNICAL FIELD

The present invention relates to a transparent tissue-visualizing preparation which is designed to be infused into or sprinkled over a transparent tissue of the eye in order to enhance visibility of transparent tissues of the eye in surgical operation, as well as to a method to visualize and enhance visibility of a transparent tissue of the eye by application of the preparation.

BACKGROUND ART

To receive light from a surrounding environment and project it on the photoreceptor cells, many portions of the eyeball are made of transparent tissues, i.e., the cornea, lens and vitreous body. The vitreous body, which adjoins the retina, provides scaffolds to proliferating tissues formed in the retina in many of retinal diseases including diabetic retinitis. As tissues that have proliferated into the vitreous body will form fibers there, which draw the retina and thereby could cause retinal detachment, a condition which, if left untreated, could eventually lead to blindness. Therefore, a surgical operation is often performed to completely remove such a vitreous body that has come to contain proliferating tissues.
In vitrectomy, it is required to remove, thoroughly as far as possible, not only proliferating tissues that have adhered to the retina but also the vitreous body, which provides scaffolds for the proliferation of such tissues. The operation is carried out while infusing into the eyeball an intraocular irrigating solution for surgical use. However, the vitreous body is a transparent tissue, whose refractive index differs very little from that of available intraocular irrigating solutions. Accordingly, when no countermeasure is taken, the transparent tissue lacks visibility in the field seen through an operation microscope, and this makes it hard to locate the tissue, thus making complete removal of it no easy task. As a means to address this problem, a steroid suspension such as a triamcinolone preparation (Kenacort-A□registered trademark) is infused during vitrectomy into a vitreous cavity, a space created by sucking and removing central part of the vitreous body, in order for letting the suspension disperse and adhere to the vitreous body and thereby visualize the vitreous body (see Non-patent Document 1). This method improves visibility of the vitreous body in the operative field, and thus makes the surgical operation easier, and complete removal of the vitreous body available. However, in association with application of a steroid preparation to the vitreous body, there have been reported, as their side effects, elevation of the intraocular pressure and progression of cataract (see Non-patent Documents 2 and 3). Thus, similar side effects could be induced by a steroid suspension used during vitrectomy for the purpose of improving visibility.
On the other hand, there is reported a method to visualize proliferating membranes and the epiretinal membrane which are associated with proliferative vitreoretinopathy (PVR), by staining them with an aqueous solution containing a dissolved dye, such as trypan blue (see Patent Document 1). An improvement in the visibility provided by the dye thus employed, however, is quite insufficient, since it darkens the operative field and, besides, does not serve to emphasize the vitreous body in the operative field.
Also in the case of cataract surgery, for example, in which the nucleus and cortex enclosed in the lens capsule are removed and then an intraocular lens is inserted, it is known that remaining cortex that could not be removed at and near the posterior capsule often proliferates and become opaque with the lapse of time after operation, thus leading to the development of postoperative cataract. Therefore, also with regard to cataract surgery, a means is needed to visualize transparent part of the cortex and thereby make its complete removal easier.

[Patent Document 1] WO 99/058159
[Non-patent Document 1] Sakamoto, T., et al., Graefe's Archive for Clinical and Experimental Ophthalmology, 240: p.423 (2002).
[Non-patent Document 2] Challa, J.K. et al., Australian and New Zealand Journal of Ophthalmology, 26: p.277 (1998)
[Non-patent Document 3] Wingate, R.J. et al., Australian and New Zealand Journal of Ophthalmology, 27: p.431 (1999)

DISCLOSURE OF INVENTION

[PROBLEM TO BE SOLVED BY THE INVENTION]

Against the above-mentioned background, the objective of the present invention is to provide a means to improve visibility of transparent tissues of the eye, i.e., vitreous body, lens or cornea in surgical operations, which means enables to achieve sufficient visibility, is easy to use and excels in safety,.

[MEANS TO SOLVE THE PROBLEM]

As a result of studies addressed to the above problem, the present inventors found that a macromolecular compound that has no pharmacological activity and dissolves in the body fluid only gradually and is eliminated and/or absorbed along with the turnover of the fluid in the eye, among others, particularly preferably, a biodegradable macromolecular compound, when infused in or sprinkled over ocular transparent tissues in the form of fine particles, adheres to the surface of those transparent tissues, on which it scatters visible light in an operative field while it remains in the form of particles, thus serving to achieve excellent visibility without raising safety concerns. The present invention has been completed through further studies based on the finding. Thus, the present invention provides what follows:

(1) A transparent tissue-visualizing preparation designed to be brought into contact with transparent tissues of the eye to enhance visibility thereof, comprising fine particles of a macromolecular compound.

(2) The transparent tissue-visualizing preparation as defined in (1) above, wherein the fine particles are those one g of which cannot completely be dissolved in less than 30 mL of water within 30 minutes at 20 °C.

(3) The transparent tissue-visualizing preparation as defined in (1) or (2) above, wherein the average size of the particles is 0.01-500 $\mu$m.

(4) The transparent tissue-visualizing preparation as defined in one of (1) to (3) above, wherein the macromolecular compound is a biodegradable macromolecular compound and/or a water soluble macromolecular compound.

(5) The transparent tissue-visualizing preparation as defined in (4) above, wherein the biodegradable macromolecular compound is selected from fatty acid polyesters, polysaccharides and derivatives thereof.

(6) The transparent tissue-visualizing preparation as defined in (4) above, wherein the water soluble macromolecular compound is selected from acrylic polymer compounds and styrene-based polymer compounds.

(7) The transparent tissue-visualizing preparation as defined in one of (1) to (3) above, wherein the macromolecular compound is selected from the group consisting of starch, soluble starch, pregelatinized starch, cellulose, acetylcellulose, hydroxypropylmethylcellulose, chitosan, chitin, dextran, polylactic acid, polyglycolic acid, lactic acid-glycolic acid copolymer, polyhydroxybutyric acid, polyhydroxyvaleric acid, polycaprolactone, hydroxybutyric acid-glycolic acid copolymer, lactic acid-caprolactone copolymer, polyethylene succinate, and polybutylene succinate, sodium polyacrylate, sodium methacrylate and sodium polystyrenesulfonate.

(8) The transparent tissue-visualizing preparation as defined in (7) above, wherein the average molecular weight of the macromolecular compound is 500-200000.

(9) The transparent tissue-visualizing preparation as defined in (8) above further comprising a polyvinyl-based compound and/or a polyol.

(10) The transparent tissue-visualizing preparation as defined in (9) above containing, per one part by weight of the fine particles, 0.05-10 part by weight of the polyvinyl-based compound and/or 0.05-10 part by weight of the polyol.

(11) The transparent tissue-visualizing preparation as defined in (9) or (10) above, wherein the polyvinyl-based compound is polyvinyl pyrrolidone and/or polyvinylalcohol.

(12) The transparent tissue-visualizing preparation as defined in (9) or (10) above, wherein the polyol is mannitol.

(13) The transparent tissue-visualizing preparation as defined in one of (1) to (12) further comprising an aqueous medium, in which the fine particles are dispersed.

(14) The transparent tissue-visualizing preparation as defined in (13) above, wherein the content of the fine particles in the transparent tissue-visualizing preparation is 0.005-10 w/v%.

(15) The transparent tissue-visualizing preparation as defined in (13) or (14) above further containing a polyvinyl-based compound and/or a polyol.

(16) The transparent tissue-visualizing preparation as defined in (15) above containing the polyvinyl-based compound at a concentration of 0.1-5 w/v% and/or the polyol at a concentration of 0.1-5 w/v%.

(17) The transparent tissue-visualizing preparation as defined in one of (1) to (16) above which is a preparation designed for injection.

(18) A transparent tissue-visualizing preparation comprising a single mixing and discharging means which encloses, separately and in a manner of keeping from contacting with each other, a powder composition comprising the fine particles as defined in one of (1) to (12) above and an aqueous medium.

(19) A method for improving visibility of a transparent tissue of the eye, which method comprises bringing a composition comprising the fine particles as defined in one of (1) to (17) into contact with a transparent tissue of the eye.

(20) Use of the fine particles as defined in one of (2) to (8) above for the production of a transparent tissue-visualizing preparation which is designed to be brought into contact with transparent tissue of the eye to visualize it.

[EFFECT OF THE INVENTION]

In surgical operations on transparent tissues of the eye, i.e., the vitreous body, the lens and the cornea, which otherwise are hardly visible in the operative field, the present invention as defined above is brought into contact with transparent tissues and greatly enhances their visibility, thereby allowing easier manipulation in such surgical operations and, therefore, steady and easier achievement of the purpose of such operations. Since fine particles having no pharmacological activity are employed, they will induce neither unnecessary pharmacological reactions nor side effects in the body. In particular, in the case where soluble and/or biodegradable fine particles are employed, even if part of the fine particles have adhered to tissues of the eye and remained there after operation, they will be eliminated from the tissues of the eye with the lapse of time through dissolution and/or decomposition, and then excretion or absorption, thus being less likely to cause a problem.

BRIEF DESCRIPTION OF DRAWINGS

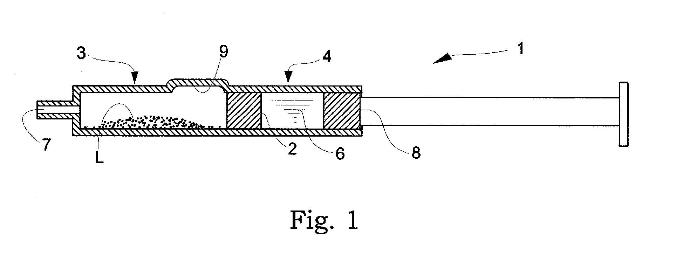

[Fig. 1] A schematic cross-sectional view of an example of the transparent tissue-visualizing preparation in the form of a prefilled double chamber syringe.

[EXPLANATION OF SIGNS]

1= double chamber syringe, 2 = slidable partition, 3 = solid phase side, 4 = liquid phase side, 7 = discharge flow path, 8 = piston, 9 = bypassing flow path

BEST MODE FOR CARRYING OUT THE INVENTION

The transparent tissue-visualizing preparation of the present invention is designed to enhance, by its infusion into or sprinkling over a transparent tissue of the eye, visibility of the transparent tissue in ophthalmic surgery. In particular, the transparent tissue-visualizing preparation of the present invention, when infused into (in the form of a liquid preparation) or sprinkled over (in the form of a powder) a vitreous cavity, countless numbers of its fine particles adhere to the vitreous gel, which, when irradiated with visible light, scatter the light along the surface of the vitreous gel and thereby enhance the visibility of the vitreous body, allowing easier visual detection of it in an operative field, e.g., through an operating microscope, during intraocular surgeries involving removal of the vitreous body for such diseases as diabetic retinopathy, retinal vein occlusion, macular edema, diabetic maculopathy, macular hole, epiretinal membrane formation, rhegmatogenous retinal detachment, and so on. Also in surgical operations which include removal of the nucleus and cortex of the lens, the preparation, when infused into or sprinkled over the lens capsule, likewise improves the visibility of them, for a countless numbers of its fine particles adhere to the nucleus and cortex of the lens and scatter light along the surface of them.
In the present invention, as a "macromolecular compound", a macromolecular compound may be employed that is pharmacologically inactive on ocular tissues and body of mammals, especially humans. There are no particular limitations as to the molecular weight of a macromolecular compound employed in the present invention, for it is enough that such a compound is solid at an ordinary temperature and can be formed into particles. Usually, the molecular weight of it is not less than 500, preferably not less than 1000, more preferably not less than 1500, and particularly preferably not less than 2000. And it is usually not more than 200000, preferably not more than 150000, more preferably not more than 100000, and particularly preferably 50000. However, those that fall outside these ranges may also be employed insofar as they serve to achieve the objective of the present invention.
Although a variety of compounds may be used as said macromolecular compound, it is preferred that one gram of fine particles made of a given macromolecular compound cannot be completely dissolved in 30 mL of water within 30 minutes at 20 °C, for such a degree of solubility is sufficient for the fine particles to resist total dissolution and remain as light scattering sources during a usual course of an surgical operation. This degree of solubility corresponds to a solubility covering from "sparingly soluble", to "slightly soluble", "very slightly soluble" and "practically insoluble" as defined in the Japanese Pharmacopoeia, 14th edition. The Pharmacopoeia defines solubility based on the extent to which one gram of a solute is dissolved in a solvent within 30 minutes at $20 \pm 5$ °C, with stirring for 30 seconds in every 5 minutes. In the present invention, this standard is substantially followed, and those fine particles are favorably employed that exhibit one of the following solubility levels when one gram of which is well stirred in water for 30 minutes at 20 °C.

(1) sparingly soluble (i.e., complete dissolution is impossible in less than 30 mL of water, but there exists a volume of water in the range of from not less than 30 mL and to less than 100 mL, in which complete dissolution is available.)
(2) slightly soluble (i.e., complete dissolution is impossible in less than 100 mL of water, but there exists a volume of water in the range of from not less than 100 mL and to less than 1000 mL, in which complete dissolution is available.)

(3) very slightly soluble (i.e., complete dissolution is impossible in less than 1000 mL of water, but there exists a volume of water in the range of from not less than 1000 mL and to less than 10000 mL, in which complete dissolution is available.)

(4) practically insoluble (i.e., at least 10000 ml of water is needed for complete dissolution).

When a macromolecular compound takes the form of a salt, preferred, but non-limiting examples include sodium salt, potassium salt, hydrochloride, etc.

There are no clear limitations as to the average size of the fine particles of a macromolecular compound. Considering, however, easy handling in use and efficiency of scattering visible light, it in general is preferably 0.01-500 $\mu$m, more preferably 0.1-200 $\mu$m, and still more preferably 1-60 $\mu$m.

Considering a possibility that the fine particles of a macromolecular compound used partly remain in the tissues after surgical operation, it is preferred that a macromolecular compound employed is biodegradable or has some solubility in water, because even if some of such fine particles are left in the tissues of the eye after a surgical operation, they will be eliminated from the tissues of the eye either along with the turnover of the liquid present in the tissues of the eye in which they are dissolved, after being decomposed with the lapse of time or without undergoing decomposition, or through absorption and further decomposition.

A variety of biodegradable macromolecular compounds may be employed in the present invention. Examples include, but not limited to, fatty acid polyesters such as polylactic acid, polyglycolic acid, lactic acid-glycolic acid copolymer, polyhydroxybutyric acid, polyhydroxyvaleric acid, polycaprolactone, hydroxybutyric acid-glycolic acid copolymer, lactic acid-caprolactone copolymer, polyethylene succinate, and polybutylene succinate and like; polysaccharides and their derivatives such as starch and starch derivatives including soluble starch, pregelatinized starch and the like, cellulose and cellulose derivatives such as acetylcellulose, hydroxypropylmethylcellulose and the like, as well as chitosan, chitin, dextran and the like.

There are no clear-cut limitations as to the level of water solubility of macromolecular compounds that may be employed in the present invention. For example, such a level of solubility is sufficient that dissolution be achieved when stirred for one week with excess amount of water at 37 °C. For, even if some portion of the fine particles are left in the eye after a surgical operation, the amount of them will usually be very small and it is without problems insofar as the particles dissolve and lose their solid form even only gradually. Examples of such macromolecular compounds, except for biodegradable macromolecular compounds, include, but are not limited to, acrylic polymers and styrene-based polymers, such as sodium polyacrylate, sodium methacrylate, and sodium polystyrenesulfonate.

The transparent tissue-visualizing preparation of the present invention may contain, along with fine particles consisting of a macromolecular compound, either one or both of polyvinyl-based compounds and polyols. Polyvinyl-based compounds and polyols act favorably to promote dispersibility of the fine particles made of a macromolecular compound. Examples of particularly preferred polyvinyl-based compounds include, but are not limited to, polyvinylpyrrolidone and polyvinylalcohol, and an example of particularly preferred polyols is mannitol. Thus, water soluble, inactive polyvinyl-based compounds and polyols that have no pharmacological activity may be employed as desired. The amount of a polyvinyl-based compound or a polyol to be employed may be usually about 0.05-10 part by weight per one part by weight of the fine particles of the macromolecular compound. However, their amount may fall outside this range, since even a lower amount of them gives some effect corresponding thereto, and higher amount of them will cause no particular disadvantage.

The transparent tissue-visualizing preparation of the present invention may be in the form of a powder, or it may be in the form comprising an aqueous medium containing fine particles of a macromolecular compound dispersed in it (i.e., suspension). In the case where the preparation is in the form of a powder, it may be used by being directly sprinkled over transparent tissues of the eye, or by being infused after mixing, prior to use, with an intraocular irrigating solution. Typically, the preparation may be used, after suspended in a conventional manner in an intraocular irrigating-washing solution (e.g., Opeguard MA (registered trademark) or Opeguard Neo Kit (registered trademark), both manufactured by Senju Pharmaceutical Co., Ltd.), or in an artificial tear or so on. If the preparation is supplied in the form of a suspension, it may be directly infused into, or applied in drops onto, transparent tissues through an injection needle.

An "aqueous medium" used for providing the transparent tissue-visualizing preparation of the present invention in the form of a suspension, is water or, as desired, a medium which is made of water and one or more additives acceptable to tissues of the eye, esp. intraocular tissues, such as isotonizers, e.g., salts and saccharides, buffering preparations, and the like. Examples of it include injectable distilled water, buffered physiological saline, commercially available intraocular irrigating solutions, and their equivalents, which may be, but is not limited to, the above-mentioned intraocular irrigating-washing solutions (e.g., Opeguard MA and Opeguard Neo Kit, both manufactured by Senju Pharmaceutical Co., Ltd.), or an artificial tear or their equivalents.

In the case where the transparent tissue-visualizing preparation of the present invention is in the form of a suspension, the content of the fine particles of a macromolecular compound in the suspension, for achieving a steady effect of enhancement of visibility, is preferably in the range of 0.005-10 w/v%, , more preferably 0.01-5 w/v%, and particularly preferably 0.1-2 w/v%. However, the content may fall outside these ranges, for a content lower than these, such as 0.0001 w/v%, would be capable of providing certain visibility, and a higher content may also be employed insofar as it causes no inconvenience in handling, e.g., in dispersing the fine particles. For convenience, the content may generally be set in the range of 0.01-5 w/v% as desired. Where one or more of the aforementioned polyvinyl-based compounds or polyols are added to increase dispersibility of the fine particles, the concentration of any of them may be set as desired in the range of 0.1-5 w/v%, though it may fall outside this range, since even a lower content would provide some effect corresponding to the content, and a higher content would bring no particular disadvantage.

The transparent tissue-visualizing preparation of the present invention may be provided, as aforementioned, in the form of a powder or, instead, a suspension consisting of aqueous medium and fine particles dispersed in it. Furthermore, it may be provided in such a form that comprises a powder consisting of fine particles of a macromolecular compound (and, as desired, one or more additives such as polyvinyl-based compounds, polyols and the like) and an aqueous medium in which to disperse the powder to make a suspension prior to use in a surgical operation, the powder and the aqueous medium being enclosed, separately and in a manner of keeping from contacting with each other, in a single mixing and discharging means. In such a case, an aqueous medium may be one of those aforementioned above.

In the present invention, there is no specific limitation as to a "mixing and discharging means" insofar as it allows mixing, by manipulation from outside, the powder and the aqueous medium separately enclosed in it, and allows discharging, by manipulation from outside, thus formed mixture to the outside. As such, there are well known a variety of two-compartment syringes. A two-compartment syringe is typically of a cylindrical body at the front end which is defined a discharge flow path around which an injection needle can be (or already is) fitted, and through the other end of which is received a liquid-tightly inserted piston, and at an intermediate region of which is placed a liquid-tightly inserted partition that is slidable in the longitudinal direction, thus forming two chambers, front and rear, within the cylindrical body. Forward of partition is provided, by forming a recess in the interior surface of the cylinder, an elongated bypassing flow path which extends in the longitudinal direction in the region greater in length than the thickness of the partition. In the front chamber is generally enclosed a dried composition, e.g., in the form of a powder, and in the rear chamber a liquid composition (e.g., a buffered solution) as a medium to be mixed with the dried composition. Mixing and discharging is performed as follows. First, the piston inserted in the rear end is forced to advance and, under a hydraulic pressure created by this, the slidable partition is then advanced forwardly to reach the central region of the bypassing flow path. The piston is forced to advance further until the liquid composition contained in the rear chamber is expelled into the front chamber through the bypassing flow path, and the contents are thus mixed within the front chamber, and then the mixture is discharged through the discharge flow path by further advancing the slidable partition through pressing the piston. It is of particular advantage to provide the transparent-visualizing preparation of the present invention in a form where the preparation is enclosed, separately and in a manner of keeping the components from contacting with each other, in such a single mixing and discharging means, for such a form greatly enhances convenience in using the preparation in a surgical operation.

The transparent tissue-visualizing preparation of the present invention, as desired, may contain: pharmaceutically acceptable additives, such as isotonizers (salts like sodium chloride, potassium chloride, etc.; saccharides like glycerol, glucose, etc.; polyols like sorbitol, mannitol, propylene glycol, etc.,; boric acid, borate, etc.), buffering agents (phosphate buffer, acetate buffer, borate buffer, carbonate buffer, citrate buffer, tris buffer, etc.), thickening agents (hydroxyethylcellulose, hydroxypropylcellulose, hydroxypropylmethylcellulose, carboxycellulose sodium, polyvinylalcohol, polyvinylpyrrolidone, polyethylene glycol, sodium alginate, etc.), stabilizers (sodium bisulfite, ascorbic acid, sodium ascorbate, dibutylhydroxytoluene, etc.), pH adjusting agents (hydrochloric acid, sodium hydroxide, phosphoric acid, acetic acid, etc.), and the like. Furthermore, insofar as it does not conflict with the purpose of the present invention, a pharmacologically active ingredient may also be contained in the transparent tissue-visualizing agent of the present invention. The transparent tissue-visualizing agent of the present invention, when provided in the form of a suspension (or in the form where a powder and an aqueous medium are separately enclosed), its pH (or pH after mixing) is generally adjusted to fall in the range of 4.0-8.0, preferably about 5.0-7.5.

The present invention also provides a method for improving visibility of a transparent tissue of the eye, which method comprises bringing a composition comprising the fine particles as described above into contact with a transparent tissue of the eye, as well as use of the fine particles as described above for the production of a transparent tissue-visualizing preparation which is designed to be brought into contact with a transparent tissue of the eye to visualize it.

EXAMPLES

The present invention will be described in further detail with reference to examples, comparison examples and test examples. However, it is not intended that the present invention be limited to those examples.

[Test Example 1] Test for visibility improvement of the vitreous body

Pigmented rabbits were euthanized and the eyeballs (6 from three rabbits) were enucleated. The cornea was excised by giving a cut with scissors along the limbus. The iris and the lens were removed to leave an optic cup behind, which was spread by giving it a crucial incision from the anterior side. Vitreous body that flowed out was removed and the retina was exposed, with vitreous body attached to part of it. An aqueous preparation (suspension) containing 1 w/v% of soluble starch was prepared according to the formula below, about 100 $\mu$l of which was applied in drops onto that part of the retina. Using an optical microscope, observations were made of the part onto which the preparation was applied in drops. As a result, fine particles of the soluble starch were found adhering to the vitreous body in all of the 6 eyes, and the visibility of the vitreous body was thereby improved.
> (Formula)
> Soluble starch    1.0 g
> Opeguard MA to    100 mL

(For Opeguard MA, see Test Materials and Reagents below)

[Test Example 2] Confirmation of visibility on enucleated pig eye

1. Tissue used:

Pig eyeballs purchased from a meat processing facility were used. Immediately after purchase, tissues surrounding the pig eyeballs were cut off and removed, and the eyeballs thus isolated were immersed in a solution of low molecular weight dextran (Low Molecular Weight Dextran L Injection, Otsuka Pharmaceutical Co., Ltd.) until the test was started.

2. Test Materials and Reagents:

The following test materials and reagents were used.

(1) Soluble starch (manufactured by Nacalai Tesque, Inc., Reagent Code 33131-42, Spec. GR (Guaranteed Reagent))
(2) Chitosan (manufactured by Funakoshi Co., Ltd., Reagent Code K-03, Particle size: 30 mesh pass -- 95% or more)
(3) PLA-0005 (manufactured by Wako Pure Chemical Industries, Ltd., D, L-polylactic acid (MW 5000))
(4) PLA-0010 (manufactured by Wako Pure Chemical Industries, Ltd., D, L-polylactic acid (MW 10000))
(5) PLGA-5005 (manufactured by Wako Pure Chemical Industries, Ltd., lactic acid-glycolic acid copolymer (MW 5000))
(6) Povidone K-30 (manufactured by BASF Japan Ltd.,. Japanese Pharmacopoeia)
(7) Gosenol EG-05 (manufactured by Nippon Synthetic Chemical Industries, Co., Ltd., polyvinylalcohol EG-05)
(8) D-mannitol (manufactured by Nacalai Tesque, Inc.)
(9) Opeguard MA (manufactured by Senju Pharmaceutical Co., Ltd., intraocular-irrigating solution, containing (per 1 mL): glucose 1.5 mg, sodium chloride 6.6 mg, potassium chloride 0.36 mg, calcium chloride 0.18 mg, magnesium sulfate 0.3 mg, sodium bicarbonate 2.1 mg)

3. Method for Preparation of Transparent Tissue-Visualizing Preparation:

3-1. Preparation of transparent tissue-visualizing preparations of Comparison Example 1 and Examples 1-5

See Table 1. PLA 0005 was gradually introduced into an A-O Jet Mill (Seishin Enterprise Co., Ltd.) and pulverized (Pressure conditions: Grinding Nozzle 0.4 MPa, Pusher Nozzle 0.4 MPa). To the powder thus obtained was added Opeguard MA to prepare 2 % and 1 % suspensions, which were made Examples 5 and 4 suspensions, respectively. The Example 4 suspension was diluted tenfold with Opeguard MA to make Example 3 suspension. The Example 3 suspension was diluted tenfold with Opeguard MA to make Example 2 suspension. The Example 2 suspension was diluted tenfold with Opeguard MA to make Comparison Example 1 suspension. Ten mL of the 1 % suspension of Example 4 was centrifuged (1000 rpm, 5 min) to spin down large particles, and the supernatant was collected to make Example 1 suspension. One mL of the supernatant was taken and, based on its dry weight, the relative content of PLA 0005 fine particles in the supernatant was determined (in this calculation, the weight of the solid originally contained in Opeguard MA was subtracted).

3-2. Preparation of transparent tissue-visualizing preparation of Comparison Examples 3-5 and Examples 9-25

See Tables 2-4. To a volume of purified water was added one of the following reagents so that the indicated concentration of it was attained, and subjected to filtration through 0.22 μm hydrophilic filter to make solution A in the corresponding example: 1.11 % of Povidone K-30 (, Examples 11-13 and 22-25, upon preparation), 1.11 % of D-mannitol (Examples 17 and 22-25, upon preparation), and 0.22 % for Gosenol EG-05 (Example 25, upon preparation). A solution of 10 % PLA0005 was prepared (acetone/ethanol = 4/6) and filtered through a 0.22 μm hydrophobic filter to make solution B. Opeguard MA was made as solution C.

The solution A for each Example and the solution B, a poly lactic acid solution, were mixed at the ratio of 9:1 in the following manner. Briefly, to the solution A, while stirred at 700-800 rpm with a stirrer, was added dropwise the solution B at a rate of about 100 μL/sec to allow PLA0005 to precipitate as fine particles. This was stirred for about 30 minutes, and aggregation product was removed through a sieve (mesh size 106 μm) to make suspension D. This, through lyophilization, gave a powder form sample E. The powder form sample E was dispersed in the solution C so that the final content of the fine particle reached 1 w/v% to make compositions of Examples 11, 17 and 22. The composition of Example 11 containing 1 w/v% of the fine particles was sequentially diluted with Opeguard MA, tenfold at a time, until a content of 0.001 % was reached, and a composition of which the content is 0.1 w/v% was made Example 10, 0.01 w/v% Example 9, and 0.001 w/v% Comparison Example 3. The composition of Example 17 was sequentially diluted in like manner with Opeguard MA, tenfold at a time, and a composition of which the content was 0.1 w/v% was made Example 16, 0.01 w/v% Example 15, 0.001 w/v% Example 14, and 0.0001 w/v% Comparison Example 4. The composition of Example 22 was sequentially diluted in like manner with Opeguard MA, tenfold at a time, and a composition of which the content was 0.1 w/v% was made Example 21, 0.01 w/v% Example 20, 0.001 w/v% Example 19, 0.0001 w/v% Example 18, and 0.00001 w/v% Comparison Example 5. The powder form sample E, which was used in Example 11, was dispersed in the solution C so that the final content of PLA0005 reached 2 w/v% or 5 w/v% to make suspensions of Examples 12 or 13, respectively. Furthermore, the powder form sample E, which was used in Example 22, was dispersed in the solution C so that the final content of PLA0005 reached 2 w/v% or 5 w/v% to make suspensions Examples 23 and 24, respectively. The powder form sample E which had been prepared through precipitation in the Gosenol EG-05-containing aqueous solution, was dispersed in the solution C so that the final content of PLA0005 was 1 w/v% to make a suspension of Example 25.

Lyophilization in the process for preparation of the compositions of Examples 11-13, Example 17 and Examples 22-25 was carried out as follows. The suspension D was frozen by storing it at -40 °C for 6 hours and, after the pressure was reduced to not higher than 100 μmHg at -40 °C, dried for at least 24 hours. The temperature then was allowed to elevate from -40 °C at a rate of 10 °C/hour until it reached +20 °C. Further drying at +20 °C at not higher than 100 μmHg for 24 hours gave the powder sample.

3-3. Preparation of transparent tissue-visualizing preparations of Comparison Example 2 and Examples 6-8:

Ten mL of the suspension of Example 11 prepared in the same manner as described in 3-2 above was centrifuged (conditions: 1000 rpm, 5 min) to spin down large particles and supernatant was collected. The supernatant was made a composition of Example 8. The composition of Example 8 was tenfold diluted with Opeguard MA to make a composition of Example 7, which in turn was tenfold diluted with Opeguard MA to make a composition of Example 6, which then was tenfold diluted with Opeguard MA to make a composition of Comparison Example 2. The content of PLA0005 in the supernatant was determined based on the dry weight of 1 mL of the supernatant of the composition of Example 8. In this calculation, the weight of Povidone and the solid contained in Opeguard MA was subtracted.

3-4. Preparation of the transparent tissue-visualizing preparations of Comparison Example 6 and Examples 26-31:

Referring to Table 5, soluble starch was gradually put into the A-O Jet Mill (Seishin Enterprise Co., Ltd.) and pulverized (Pressure conditions: Grinding Nozzle 0.4 MPa, Pusher Nozzle 0.4 MPa). To this sample was added Opeguard MA to prepare a suspension whose content of the fine particles was 10 w/v%, which was made a suspension of Example 31. The suspension of Example 31 was diluted with Opeguard MA, and a 1 w/v% suspension thus prepared was made Example 30, a 0.1 w/v% suspension Example 29, a 0.01 w/v% suspension Example 28, a 0.005 w/v% suspension Example 27, a 0.0025 w/v% suspension Example 26, and a 0.001 w/v% suspension Comparison Example 6.

3-5. Preparation of transparent tissue-visualizing preparations of Example 32-35:

Referring to Table 6, PLGA5005 or chitosan was gradually put into the A-O Jet Mill (Seishin Enterprise Co., Ltd.) and pulverized (Pressure conditions: Grinding Nozzle 0.4 MPa, Pusher Nozzle 0.4 MPa). To each these was added Opeguard MA to prepare 1 w/v% suspensions, and the PLGA5005 containing suspension was made Example 33, and the chitosan-containing suspension was made Example 35. And a 1 w/v% PLA0010 suspension was prepared by addition of Opeguard and made Example 34. The suspension of Example 33 was centrifuged (conditions: 1000 rpm, 5 min) to spin down large particles and supernatant was collected. The supernatant was made Example 32. The content of PLGA5005 in the supernatant was determined based on the dry weight of 1 mL of the supernatant. In this calculation, the weight of the solid contained in Opeguard MA was subtracted.

4. Test Method:

The average size of the fine particles in Example 1, Example 4, Example 8, Example 11, Example 17, Example 22, Example 30 and Examples 32-35 was measured using a laser diffraction particle size analyzer (SALD-2100, Shimadzu Corporation). The average size of the fine particles was defined as the average value that was calculated by the method shown in Note 1. Briefly, over the full range of the particle size distribution, the size of the particles falling within each of the intervals ($x_j$, $x_{j+1}$) created by dividing the range by a large number (n) was represented by the mean (in logarithm) of the logarithms of the particle sizes at the both end of the interval, and each of them were multiplied by the % difference in volume of all the particles detected in the very interval relative to the volume of those found in the entire range of distribution (i.e., frequency in the volume distribution), and the values thus obtained with regard to all the intervals, which together covered the entire range of distribution, were added and the average was taken, and, from the average ($\mu$), the average value of the size ($10\mu$) of the fine particles was calculated. In the case of Examples where dispersion of fine particles by shaking was found difficult due to their aggregation, measurement was made after they were ultrasonically dispersed. For other Comparison Examples and Examples than those describe above, no measurement of the particle size was made, since the powders used in them were of the same lots as those employed in the above described samples.

(Note 1) Method for calculation of the average particle size:

$$\mu = \frac{1}{100} \sum_{j=1}^{n} q_j \frac{\log_{10} x_j + \log_{10} x_{j+1}}{2}$$

[MATH 1]

where $x_j$: particle size ($\mu$m)
$q_j$: % difference in volume (frequency in the volume distribution)

For performing vitreous surgery, a surgical instrument for vitreous surgery (Ocutome (registered trademark), ALCON) and a microscope for ophthalmic surgery (CARL ZEISS) were used. A pig eyeball was incised at a point outside the limbus, about 3 mm away from it, with a knife for ophthalmic surgery (20G V-Lance™□ALCON), and the sclera was cut parallel to the limbus to create a port for an irrigation cannula. An irrigation cannula was inserted through the port thus created, and physiological saline (Otsuka Pharmaceutical Co., Ltd.) was constantly infused during the surgical operation. With a knife for ophthalmic surgery (20G V-Lance™□ALCON), an incision parallel to the limbus was made to the sclera at a point about 3 mm outside of the limbus and at about 120 °, around the center of the cornea, away from the intraocular irrigation port, to create a port for vitreous cutter. A vitreous cutter was inserted through the port thus created, and the lens and other tissues surrounding it were removed and, after it was confirmed through a microscope that the ocular fundus was visible, a central portion of the vitreous body was removed. Using a disposable syringe (5 mL Terumo Corporation) equipped with a non bevel needle (22G, Terumo Corporation), 1 mL of a transparent tissue-visualizing preparation was infused, through the port for a vitreous cutter, into the cavity which had been created by removal of the central portion of the vitreous body. After the fine particles drifting in the liquid was removed by suctioning with the vitreous cutter, a light guide was inserted through the port for a vitreous cutter and the visibility of the vitreous body due to the adhered fine particles were assessed by examining the anterior part (lenticular side), equatorial part and the posterior part of the vitreous body (retinal fundus side), respectively, according to the following criterion. Transparent tissue-visualizing preparations were assessed for their visualizing ability/inability, as well as the degree of it, and judged to possess a practical level of visualizing effect if score 2 or 3 of the assessment criterion table was obtained with at least one of these parts.

< Assessment Criterion >

0 No fine particle was observed adhering to the remaining vitreous body, and the shape of the vitreous body is not visible, either.

1 Fine particles were observed adhering to the remaining vitreous body, but the shape of the vitreous body is not visible.

2 The shape of the remaining vitreous body is visible due to adhering fine particles, with the retina clearly visible through the adhering fine particles.

3 The shape of the remaining vitreous body is visible due to adhering fine particles, with the retina only dimly visible through the adhering particles.

5. Test Results:

5-1. Visualizing ability of PLA-0005-containing transparent tissue-visualizing preparations:

The results of the test on visibility improving effect of PLA-0005-containing transparent tissue-visualizing preparations are shown in Tables 1-4. To prepare PLA0005 of different particle sizes, PLA0005 was pulverized using the A-O Jet Mill for Comparison Example 1 and Examples 1-5. For Comparison Examples 2-5 and Examples 6-25, fine particles were prepared by dissolving PLA0005 in an organic solvent and then causing precipitation to take place as fine particles. PLA0005 was precipitated as fine particles in a Povidone solution for Comparison Examples 2 and 3 and Examples 6-13, in a mannitol solution for Comparative Example 4 and Examples 14-17, in a solution of Povidone and mannitol for Comparative Example 5 and Examples 18-24, and in a solution of Gosenol and mannitol for Example 25. For Comparative Example 2 and Example 1 and Examples 6-8, a high content PLA0005 suspension was centrifuged and supernatant was collected to obtain finer particles. As a result of these processes, fine particles were obtained whose average particle size fell in the range of 0.8 $\mu$m-60.3 $\mu$m. For these fine particles, concentrations giving useful vitreous body-visualizing preparations were determined in the test of a visualization effect, and, as a result, it was found that the fine particles that exhibited a visualization effect at the lowest content was those of PLA0005 of an average particle size of 17.9 $\mu$m, which was useful down to the content as low as 0.0001 w/v% (Example 18). PLA0005 particles of the smallest particle size of 0.8 $\mu$m of the samples were found useful down to the content as low as 0.0073 w/v% (Example 6). PLA0005 particles of the largest size of 60.3 $\mu$m were found useful down to the content as low as 0.001 % (Example 14). Particles of the sizes of 17.9 and 22 $\mu$m were found to be dispersed even at a content of as high as 5 %, with good visualizing effect, thus were found useful as transparent tissue-visualizing preparations (Example 13 and Example 24). When the content of PLA0005 was 2 %, particles of any of the sizes tested were found usable as transparent tissue-visualizing preparations (Example 5, Example 12 and Example 23). From the findings as noted above, it is evident that the tested polylactic acid fine particles could exhibit a visibility improving effect even at a content of 0.0001 w/v%, and will unfailingly exhibit the effect at a content of not less than 0.005 w/v%.

Table 1. Results of test on visibility improving effect of PLA-0005-containing transparent tissue-visualizing preparations

| | | Example 1 | Comparison Example 1 | Example 2 | Example 3 | Example 4 | Example 5 | Comparison Example 2 | Example 6 |
|---|---|---|---|---|---|---|---|---|---|
| PLA-0005 (g) | | 0.36 | 0.001 | 0.01 | 0.1 | 1 | 2 | 0.00073 | 0.0073 |
| Povidone (g) | | - | - | - | - | - | - | 0.00073 | 0.0073 |
| D-Mannitol (g) | | - | - | - | - | - | - | - | - |
| Opeguard MA (to 100 mL) | | q.s. | q.s. | q.s. | q.s. | q.s. | q.s. | q.s. | q.s. |
| Average particle size (μm) | | 1.9 | 6.2 | 6.2 | 6.2 | 6.2 | 6.2 | 0.8 | 0.8 |
| Visibility | Anterior part | 2 | 1 | 2 | 2 | 3 | 3 | 1 | 2 |
| | Posterior part | 1 | 1 | 1 | 2 | 3 | 3 | 1 | 2 |
| | Equatori ·al part | 2 | 1 | 2 | 2 | 3 | 3 | 1 | 2 |
| | Sum | 5 | 3 | 5 | 6 | 9 | 9 | 3 | 6 |

Table 2. Results of test on visibility improving effect of PLA-0005-containing transparent tissue-visualizing preparations

| | | Example 7 | Example 8 | Comparison Example 3 | Example 9 | Example 10 | Example 11 | Example 12 | Example 13 |
|---|---|---|---|---|---|---|---|---|---|
| PLA-0005 (g) | | 0.073 | 0.73 | 0.001 | 0.01 | 0.1 | 1 | 2 | 5 |
| Povidone (g) | | 0.073 | 0.73 | 0.001 | 0.01 | 0.1 | 1 | 2 | 5 |
| D-Mannitol (g) | | - | - | - | - | - | - | - | - |
| Opeguard MA (to 100 mL) | | q.s. | q.s. | q.s. | q.s. | q.s. | q.s. | q.s. | q.s. |
| Average particle size (μm) | | 0.8 | 0.8 | 22 | 22 | 22 | 22 | 22 | 22 |
| Visibility | Anterior part | 2 | 3 | 1 | 2 | 3 | 3 | 3 | 3 |
| | Posterior part | 2 | 3 | 1 | 2 | 3 | 3 | 3 | 3 |
| | Equatori-al part | 2 | 3 | 1 | 2 | 3 | 3 | 3 | 3 |
| | Sum | 6 | 9 | 3 | 6 | 9 | 9 | 9 | 9 |

Table 3. Results of test on visibility improving effect of PLA-0005-containing transparent tissue-visualizing preparations

| | | Comparison Example 4 | Example 14 | Example 15 | Example 16 | Example 17 | Comparison Example 5 | Example 18 | Example 19 |
|---|---|---|---|---|---|---|---|---|---|
| PLA-0005 (g) | | 0.0001 | 0.001 | 0.01 | 0.1 | 1 | 0.00001 | 0.0001 | 0.001 |
| Povidone (g) | | - | - | - | - | - | 0.00001 | 0.0001 | 0.001 |
| D-Mannitol (g) | | 0.0001 | 0.001 | 0.01 | 0.1 | 1 | 0.00001 | 0.0001 | 0.001 |
| Opeguard MA (to 100 mL) | | q.s. | q.s. | q.s. | q.s. | q.s. | q.s. | q.s. | q.s. |
| Average particle size (μm) | | 60.3 | 60.3 | 60.3 | 60.3 | 60.3 | 17.9 | 17.9 | 17.9 |
| Visibility | Anterior part | 1 | 2 | 2 | 2 | 3 | 1 | 2 | 2 |
| | Posterior part | 0 | 1 | 2 | 2 | 3 | 0 | 1 | 1 |
| | Equatori-al part | 1 | 2 | 2 | 2 | 3 | 1 | 2 | 2 |
| | Sum | 2 | 5 | 6 | 6 | 9 | 2 | 5 | 5 |

Table 4. Results of test on visibility improving effect of PLA-0005-containing transparent tissue-visualizing preparations

| | Example 20 | Example 21 | Example 22 | Example 23 | Example 24 | Example 25 |
|---|---|---|---|---|---|---|
| PLA-0005 (g) | 0.01 | 0.1 | 1 | 2 | 5 | 1 |
| Povidone (g) | 0.01 | 0.1 | 1 | 2 | 5 | - |
| Gosenol (g) | - | - | - | - | - | 0.2 |
| D-Mannitol (g) | 0.01 | 0.1 | 1 | 2 | 5 | 1 |
| Opeguard MA (to 100 mL) | q.s. | q.s. | q.s. | q.s. | q.s. | q.s. |
| Average particle size (μm) | 17.9 | 17.9 | 17.9 | 17.9 | 17.9 | 11.9 |
| Visibility — Anterior part | 2 | 2 | 3 | 3 | 3 | 3 |
| Visibility — Posterior part | 1 | 2 | 3 | 3 | 3 | 3 |
| Visibility — Equatori-al part | 2 | 2 | 3 | 3 | 3 | 3 |
| Visibility — Sum | 5 | 6 | 9 | 9 | 9 | 9 |

5-2. Results of test on visibility improving effect of soluble starch-containing transparent tissue-visualizing preparations

Referring to Table 5, soluble starch was pulverized using A-O Jet Mill to obtain particles of the size of 35.8 μm. The 35.8 μm particles successfully visualized vitreous body at the content down to 0.0025 w/v% (Example 26). Therefore, they can be used as a transparent tissue-visualizing preparation at a content falling in the range of at least from 0.0025 % to 10 w/v%.
Table 5. Results of test on visibility improving effect of soluble starch-containing transparent tissue-visualizing preparations

| | Comparison Example 6 | Example 26 | Example 27 | Example 28 | Example 29 | Example 30 | Example 31 |
|---|---|---|---|---|---|---|---|
| Soluble starch (g) | 0.001 | 0.0025 | 0.005 | 0.01 | 0.1 | 1 | 10 |
| Opeguard MA (to 100 mL) | q.s. | q.s. | q.s. | q.s. | q.s. | q.s. | q.s. |
| Average particle size (μm) | 35.8 | 35.8 | 35.8 | 35.8 | 35.8 | 35.8 | 35.8 |
| Visibility — Anterior part | 1 | 2 | 2 | 1 | 2 | 2 | 3 |
| Visibility — Posterior part | 1 | 1 | 1 | 2 | 2 | 3 | 3 |
| Visibility — Equatori-al part | 1 | 1 | 2 | 1 | 2 | 2 | 3 |
| Visibility — Sum | 3 | 4 | 5 | 4 | 6 | 7 | 9 |

5-3. Results of test on visibility improving effect of transparent tissue-visualizing preparations containing other macromolecular compounds

Referring to Table 6, vitreous body was made visible with the use of the fine particles consisting of PLGA5005, PLA0010 or chitosan, at a content of 1 w/v% (Examples 33-35). PLGA5005 at a content of 0.01 % could also visualize the vitreous body (Example 32).
Table 6. Results of test on visibility improving effect of transparent tissue-visualizing preparations containing other macromolecular compounds

|  |  | Example 32 | Example 33 | Example 34 | Example 35 |
|---|---|---|---|---|---|
| PLGA-5005 (g) |  | 0.01 | 1 | - | - |
| PLA-0010 (g) |  | - | - | 1 | - |
| Chitosan (g) |  | - | - | - | 1 |
| Opeguard MA (to 100 mL) |  | q.s. | q.s. | q.s. | q.s. |
| Average particle size (μm) |  | 3.1 | 15.5 | 65.4 | 133.9 |
| Visibility | Anterior part | 2 | 3 | 2 | 3 |
|  | Posterior part | 2 | 3 | 2 | 3 |
|  | Equatorial part | 2 | 3 | 2 | 3 |
|  | Sum | 6 | 9 | 6 | 9 |

[Test Example 2] Test of redispersibility of transparent tissue-visualizing preparations

1. Test Method:

See Table 7. Transparent tissue-visualizing preparations of Examples 36-38 were prepared in the same manner as in Test Example 1, In a 5-mL syringe, 0.02 g of the powder sample prepared in Example 36, 0.04 g of the powder sample prepared in Example 37 or 0.06 g of the powder sample prepared in Example 38 was mixed with 2 mL of the following dispersion medium and shaken by hand, and the number of the shakes that was required for the powder to be dispersed in the dispersion medium was counted.

<Formula of dispersion medium>
Sodium chloride               0.75 g
Potassium chloride            0.16 g
Sodium hydrogen phosphate     0.1 g
Sodium dihydrogen phosphate   0.015 g
Hydrochloric acid             q.s. (pH 7)
Purified water                to 100 mL

Table 7. Redispersibility of preparations containing Povidone and D-mannitol

| | Example 36 | Example 37 | Example 38 |
|---|---|---|---|
| PLA-0005 (g) | 1 | 1 | 1 |
| D-Mannitol (g) | - | 1 | 1 |
| Povidone (g) | - | - | 1 |
| Dispersion medium* (to 100 mL) | q.s. | q.s. | q.s. |
| Number of shakes needed until the powder was dispersed | 200 or more | 15 | 7 |

\* A phosphate buffer (pH 7) was employed containing 0.75 % of sodium chloride, and 0.16 % of potassium chloride.

2. Test Results:

The number of shakes required to achieve dispersion of the PLA0005 fine particles after their mixing with the medium was reduced by addition of mannitol, indicating that redispersibility was improved. When Povidone was further added to the mixture, the number of shakes required to achieve dispersion was further reduced, showing that further improvement in redispersibility was obtained.

[Production Example 1] Prefilled vial-type transparent tissue-visualizing preparation

An aqueous solution containing 1.11 w/v% of D-mannitol and 1.11 w/v% of Povidone K-30 was filtered through a hydrophilic filter having the pore size of 0.22 $\mu$m to make a solution A. An acetone solution of 10 w/v% of PLA0005 (acetone/ethanol = 4/6) was prepared and filtered through a hydrophobic filter having the pore size of 0.22 $\mu$m to make a solution B. Opeguard MA was made a solution C.
The solutions A and B were mixed at a proportion of 9:1 in the following manner. Briefly, to the solution A, stirred at 700-800 rpm with a stirrer, was added the solution B, at a rate of about 100 $\mu$L/sec, to let PLA0005 precipitate as fine particles. The mixture was stirred for about 30 minutes, and aggregation products were removed through a sieve (mesh size 106 $\mu$m) to obtain a suspension D. Lyophilization of this in a vial gave a powder form sample E. Prior to use, the solution C was poured into the vial containing the powder form sample E to prepare a sample.
Lyophilization was carried out in the following manner. Briefly, the suspension D was frozen by storing it at -40 °C for 6 hours and then, after the pressure was reduced to 100 $\mu$mHg at -40 °C, dried for at least 24 hours. The temperature then was allowed to elevate from -40 °C, at a rate of 10 °C/hour, until it reached +20 °C. The drying process was further continued at +20 °C and below 100 $\mu$mHg for at least 24 hours.

[Production Example 2] Prefilled double chamber syringe-type transparent tissue-visualizing preparation

An aqueous solution containing 1.11 w/v% of D-mannitol and 1.11 w/v% of Povidone K-30 was filtered through a hydrophilic filter having the pore size of 0.22-$\mu$m to make a solution F. On the other hand, a 10 w/v% solution of PLA0005 (acetone/ethanol = 4/6) was filtered through a hydrophobic filter having the pore size of 0.22 $\mu$m to make a solution G. A phosphate buffer (pH 7) containing 0.75 % of sodium chloride and 0.16 % of potassium chloride was made a liquid H.

The solutions F and G were mixed at a proportion of 9:1 in the following manner. Briefly, to the solution F, stirred at 700-800 rpm with a stirrer, was added the solution G, at a rate of about 100 μL/sec, to allow PLA0005 to precipitate as fine particles. The mixture was stirred for about 40 minutes (for 10 minutes of which a reduced pressure was applied), and aggregation products were removed through a sieve (mesh size 106 μm) to obtain a suspension J. Two ml each of this was dispensed on the solid phase side 3 in the double chamber syringe 1, which is schematically illustrated in Fig. 1, and subjected to lyophilization to give powder L. By fitting a rubber plug, which served as the slidable partition 2 between the solid phase side 3 and the liquid phase side 4, filling the syringe, on the liquid phase side 4, with 2 mL of the liquid H serving as the medium 6, and then fitting a plug serving as the piston 8, a prefilled double chamber syringe-type transparent tissue-visualizing preparation was obtained. The numeral 9 indicates a longitudinal bypassing flow path, which is defined by a partial recess in the interior wall of the double chamber syringe 1. Lyophilization was carried out in the following manner. Briefly, the suspension J was frozen by storing it at -40 °C for 6 hours and, after the pressure was reduced to 100 μmHg at -40 °C, dried for at least 24 hours. The temperature then was allowed to elevate from -40 °C, at a rate of 10 °C/hour, until it reached +20 °C. The drying process was further continued at +10 °C and below 100 μmHg for at least 24 hours.

The transparent tissue-visualizing preparation of this example is used in the following manner. Briefly, the piston 8 is pushed in to advance, and, utilizing the pressure generated by this in the medium liquid H enclosed on the liquid phase side 4, the slidable partition 2 then is pushed to advance. When the rear edge of the slidable partition 2 reaches the bypassing flow path 9, the liquid phase side 4 and the solid phase side 3 are placed into communication with each other, and the liquid H starts to flow into the solid side 3. By further pushing in the piston 8 until it abuts on the slidable partition 2, all the liquid H is transferred to the solid phase side 3, where it mixes with the powder L. After completion of the mixing, the piston 8 is further pushed in (together with the slidable partition 2) to advance, and the mixture liquid then is discharged from the discharge flow path 7 (through a needle, etc. not shown) to the part on which a surgical operation is being performed.

[Preparation Example 1] Transparent tissue-visualizing preparation containing soluble starch fine particles

A transparent tissue-visualizing preparation of the following formula was prepared in a conventional manner. In the preparation, the average particle size of the soluble starch fine particles was about 50 μm.

| | |
|---|---|
| Soluble starch | 1.0 g |
| Opeguard MA | to 100 mL |

[Preparation Example 2] Transparent tissue-visualizing preparation containing chitosan fine particles

A transparent tissue-visualizing preparation of the following formula was prepared in a conventional manner.

| | |
|---|---|
| Chitosan | 1.0 g |
| Opeguard MA | to 100 mL |

[Preparation Example 3] Transparent tissue-visualizing preparation containing polylactic acid fine particles

A transparent tissue-visualizing preparation of the following formula was prepared in a conventional manner. In the preparation, the average particle size of the polylactic acid fine particles was about 0.2-0.3 μm.

| | |
|---|---|
| Polylactic acid | 1.0 g |
| Opeguard MA | to 100 mL |

[Preparation Example 4] Transparent tissue-visualizing preparation containing lactic acid-glycolic acid copolymer fine particles

A transparent tissue-visualizing preparation of the following formula was prepared in a conventional manner. In the preparation, the average particle size of the lactic acid-glycolic acid copolymer fine particles was about 0.06-0.07 μm.

| | |
|---|---|
| Lactic acid-glycolic acid copolymer | 1.0 g |

(continued)

| Opeguard MA | to 100 mL |

[Preparation Example 5] Transparent tissue-visualizing preparation containing soluble starch fine particles

A transparent tissue-visualizing preparation of the following formula was prepared in a conventional manner.

| Soluble starch | 1.0 g |
| Sodium dihydrogen phosphate, dihydrate | 0.10 g |
| Sodium chloride | 0.9 g |
| Sodium hydroxide | q.s. |
| Purified water | to 100 mL |
| pH | 7.0 |

[Preparation Example 6] Transparent tissue-visualizing preparation containing chitosan fine particles

A transparent tissue-visualizing preparation of the following formula was prepared in a conventional manner.

| Chitosan | 0.10 g |
| Sodium chloride | 0.55 g |
| Potassium chloride | 0.16 g |
| Exsiccated sodium carbonate | 0.06 g |
| Sodium hydrogen phosphate | 0.18 g |
| Boric acid | 1.2 g |
| Borax | q.s. |
| Sterile purified water | to 100 mL |
| pH | 7.3 |

[Preparation Example 7] Transparent tissue-visualizing preparation containing polylactic acid fine particles

A transparent tissue-visualizing preparation of the following formula was prepared in a conventional manner.

| Polylactic acid | 10 g |
| Sodium dihydrogen phosphate, dihydrate | 0.1 g |
| Sodium chloride | 0.9 g |
| Sodium hydroxide | q.s. |
| Sodium edetate | 0.1 g |
| Sterile purified water | to 100 mL |
| pH | 7.0 |

[Preparation Example 8] Transparent tissue-visualizing preparation containing lactic acid-glycolic acid copolymer fine particles

A transparent tissue-visualizing preparation of the following formula was prepared in a conventional manner.

| Lactic acid-glycolic acid copolymer | 0.10 g |
| Sodium chloride | 0.55 g |
| Potassium chloride | 0.16 g |
| Exsiccated sodium carbonate | 0.06 g |
| Sodium hydrogen phosphate | 0.18 g |
| Boric acid | 1.2 g |
| Borax | q.s. |

(continued)

| | |
|---|---|
| Hydroxypropylmethylcellulose | 0.1 g |
| Sterile purified water | to 100 mL |
| pH | 7.3 |

INDUSTRIAL APPLICABILITY

In surgical operations on transparent tissues of the eye, i.e., the vitreous body, the lens and the cornea, the present invention as defined above greatly enhances visibility of transparent tissues, which otherwise are hardly visible in the operative field, neither causing unnecessary pharmacological reactions nor side effects in the body, thereby allowing easier manipulation in such surgical operations and, therefore, making it easier to unfailingly achieve the purpose of the such surgical operations.

**Claims**

1. A transparent tissue-visualizing preparation designed to be brought into contact with transparent tissues of the eye to enhance visibility thereof, comprising fine particles of a macromolecular compound.

2. The transparent tissue-visualizing preparation of claim 1, wherein the fine particles are those one g of which cannot completely be dissolved in less than 30 mL of water within 30 minutes at 20 °C.

3. The transparent tissue-visualizing preparation of claim 1 or 2, wherein the average size of the particles is 0.01-500 $\mu$m.

4. The transparent tissue-visualizing preparation of one of claims 1 to 3, wherein the macromolecular compound is a biodegradable macromolecular compound and/or a water soluble macromolecular compound.

5. The transparent tissue-visualizing preparation of claim 4, wherein the biodegradable macromolecular compound is selected from fatty acid polyesters, polysaccharides and derivatives thereof.

6. The transparent tissue-visualizing preparation of claim 4, wherein the water soluble macromolecular compound is selected from acrylic polymer compounds and styrene-based polymer compounds.

7. The transparent tissue-visualizing preparation of one of claims 1 to 3, wherein the macromolecular compound is selected from the group consisting of starch, soluble starch, pregelatinized starch, cellulose, acetylcellulose, hydroxypropylmethylcellulose, chitosan, chitin, dextran, polylactic acid, polyglycolic acid, lactic acid-glycolic acid copolymer, polyhydroxybutyric acid, polyhydroxyvaleric acid, polycaprolactone, hydroxybutyric acid-glycolic acid copolymer, lactic acid-caprolactone copolymer, polyethylene succinate, and polybutylene succinate, sodium polyacrylate, sodium methacrylate and sodium polystyrenesulfonate.

8. The transparent tissue-visualizing preparation of claim 7, wherein the average molecular weight of the macromolecular compound is 500-200000.

9. The transparent tissue-visualizing preparation of claim 8 further comprising a polyvinyl-based compound and/or a polyol.

10. The transparent tissue-visualizing preparation of claim 9 containing, per one part by weight of the fine particles, 0.05-10 part by weight of the polyvinyl-based compound and/or 0.05-10 part by weight of the polyol.

11. The transparent tissue-visualizing preparation of claim 9 or 10, wherein the polyvinyl-based compound is polyvinyl pyrrolidone and/or polyvinylalcohol.

12. The transparent tissue-visualizing preparation of claim 9 or 10, wherein the polyol is mannitol.

13. The transparent tissue-visualizing preparation of one of claims 1 to 12 further comprising an aqueous medium, in which the fine particles are dispersed.

**14.** The transparent tissue-visualizing preparation of claim 13, wherein the content of the fine particles in the transparent tissue-visualizing preparation is 0.005-10 w/v%.

**15.** The transparent tissue-visualizing preparation of claim 13 or 14 further containing a polyvinyl-based compound and/or a polyol.

**16.** The transparent tissue-visualizing preparation of claim 15 containing the polyvinyl-based compound at a concentration of 0.1-5 w/v% and/or the polyol at a concentration of 0.1-5 w/v%.

**17.** The transparent tissue-visualizing preparation of one of claims 1 to 16 which is a preparation designed for injection.

**18.** A transparent tissue-visualizing preparation comprising a single mixing and discharging means which encloses, separately and in a manner of keeping from contacting with each other, a powder composition comprising the fine particles of one of claims 1 to 12 and an aqueous medium.

**19.** A method for improving visibility of a transparent tissue of the eye, which method comprises bringing a composition comprising the fine particles of one of claims 1 to 17 into contact with a transparent tissue of the eye.

**20.** Use of the fine particles of one of claims 2 to 8 for the production of a transparent tissue-visualizing preparation which is designed to be brought into contact with transparent tissue of the eye to visualize it.

Fig. 1

## INTERNATIONAL SEARCH REPORT

| International application No. |
| --- |
| PCT/JP2005/009846 |

| A. CLASSIFICATION OF SUBJECT MATTER |
| --- |
| Int.Cl$^7$  A61K31/718, 31/722, 31/765, 45/00, A61P27/02, 27/12 |

According to International Patent Classification (IPC) or to both national classification and IPC

| B. FIELDS SEARCHED |
| --- |
| Minimum documentation searched (classification system followed by classification symbols) |
| Int.Cl$^7$  A61K31/718, 31/722, 31/765, 45/00, A61P27/02, 27/12 |

Documentation searched other than minimum documentation to the extent that such documents are included in the fields searched

| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CA(STN)

| C. DOCUMENTS CONSIDERED TO BE RELEVANT |
| --- |

| Category* | Citation of document, with indication, where appropriate, of the relevant passages | Relevant to claim No. |
| --- | --- | --- |
| A | JP 2002-514471 A  (Melles, Gerrit Reinold Jacob),<br>21 May, 2002 (21.05.02),<br>Full text<br>& EP 963759 A           & EP 1075285 B1<br>& US 6720314 B1          & WO 99/58160 A | 1-18,20 |
| A | JP 2003-504118 A  (CORONEO, Minas, Theodore),<br>04 February, 2003 (04.02.03),<br>Full text<br>& WO 01/003620 A | 1-18,20 |

☒  Further documents are listed in the continuation of Box C.          ☐  See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 02 August, 2005 (02.08.05) | 16 August, 2005 (16.08.05) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

**INTERNATIONAL SEARCH REPORT**

| International application No. |
| --- |
| PCT/JP2005/009846 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2003-525975 A  (Melles, Gerrit Reinold Jacob), 02 September, 2003 (02.09.03), Full text & WO 01/066053 A        & EP 1132065 A & EP 1263363 A        & US 2003/088233 A | 1-18,20 |
| A | WO 02/036096 A  (Alcon, Inc.), 10 May, 2002 (10.05.02), Full text & JP 2004-530452 A      & CA 2428066 A & EP 1341519 A        & US 2003/060447 A | 1-18,20 |
| A | JP 2002-136588 A  (Kuraray Co., Ltd.), 14 May, 2002 (14.05.02), Full text (Family: none) | 1-18,20 |
| A | Suresh K. Pandey, MD et al., Dye-enhanced cataract surgery, J. Cataract Research Surg., Vol.26, 2000, pages 1066 to 1071 | 1-18,20 |

Form PCT/ISA/210 (continuation of second sheet) (January 2004)

### INTERNATIONAL SEARCH REPORT

| International application No. |
| --- |
| PCT/JP2005/009846 |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 19
   because they relate to subject matter not required to be searched by this Authority, namely:
   Claim 19 pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest.

☐ No protest accompanied the payment of additional search fees.

Form PCT/ISA/210 (continuation of first sheet (2)) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 99058159 A

**Non-patent literature cited in the description**

- **SAKAMOTO, T. et al.** *Graefe's Archive for Clinical and Experimental Ophthalmology,* 2002, vol. 240, 423
- **CHALLA, J.K. et al.** *Australian and New Zealand Journal of Ophthalmology,* 1998, vol. 26, 277
- **WINGATE, R.J. et al.** *Australian and New Zealand Journal of Ophthalmology,* 1999, vol. 27, 431